# EUROPEAN PATENT APPLICATION

(11) **EP 4 617 277 A1**
(43) Date of publication of application: **17.09.2025**
(21) Application number: 24162930.2
(22) Date of filing: 12.03.2024
(51) Int. Cl.: C07F 9/6518, C07D 249/12, C07D 251/54, C07G 1/00, C08L 97/00, C09K 21/14

(54) **TAD-MODIFICATION OF LIGNIN FOR IMPROVED FIRE-RETARDANT PROPERTIES**

(71) Applicant: VITO NV, 2400 Mol (BE)
(72) Inventor: NAHRA, Fady, 2400 Mol (BE); DOAN, Thu Hong, 2400 Mol (BE); COMI BONACHI, Marc, 2400 Mol (BE); RUBENS, Maarten, 2400 Mol (BE); FEGHALI, Elias, 2400 Mol (BE)
(74) Representative: Arnold & Siedsma

(57) **Abstract**

A novel modification of lignin which improves its flame-retardant property is described. This functionalization involves an insertion of TAD (1,2,4-triazoline-3,5-dione) moiety to lignin system. The TAD derivatives, bearing a nitrogen-rich skeleton, were chosen thanks to their ability to release non-flammable gases to dilute fuel and cool down a burning area as well as form a barrier to prevent heat transfer.

## Description

### Field of the invention

A novel modification of lignin which improves its flame-retardant property is described. This functionalization involves an insertion of TAD (1,2,4-triazoline-3,5-dione) moiety to lignin system. The TAD derivatives, bearing a nitrogen-rich skeleton, were chosen thanks to their ability to release non-flammable gases to dilute fuel and cool down a burning area as well as form a barrier to prevent heat transfer.

### Background

Lignin is the second most abundant natural polymer on earth, accounting for 15-30% of the earth's biomass. Native lignins are complex heterogeneous aromatic polymers stemming from the radical polymerization of three aromatic alcohols called monolignol units: p-coumaryl, confineryl, and sinapyl alcohols. Depending on the type of lignocellulosic species the proportions of monolignols can vastly differ. The individual monolignols are bonded by different bonding motifs, with β-O-4 ether linkages being the predominant linker type. Lignin is the most sustainable source of aromatic compounds, but its use is limited to only specialty products.

Lignin does not have a defined structure, and its structure depends not only on its botanical source but also on the applied isolation and post-treatment processes. In addition, depolymerization or fractionation of the lignin yields mixtures with increased content in monomers, dimers, and low molecular weight oligomers. Depending on all these factors, the molecular weight and amount of aromatic and aliphatic hydroxyl groups can vastly differ. However, it will always have a repeating unit represented by formula (i-a) or (i-b) or comprise monomer represented by formula (ii) derived from the monolignols it is synthesized from, or repeating units derived from other link breakage, or depolymerized fragments represented by formula (iii).

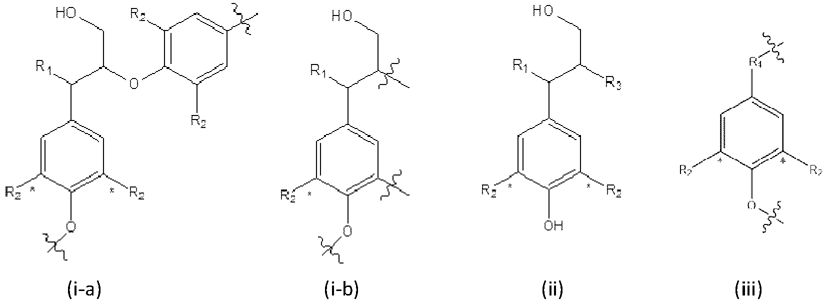

Wherein R₁: H or OSO₃M or SH
R₂: H or OCH₃
R₃: H, O-alkyl, or optionally substituted O-aryl
R₄: a direct bond, C₁₋₆alkyl, or C₂₋₆alkenyl, wherein each of said C₁₋₆alkyl, and C₂₋
₆alkenyl is independently and optionally substituted with from 1 to 3 substituents selected from OH, OSO₃M, SH and O-C₁₋₆alkyl.
M: alkaline metal
* Indicates the ortho position vis-à-vis the phenolic OH of the lignin.

The most common monomers derived from the monolignols can be represented by formulas (ii-a) and (ii-b) below.

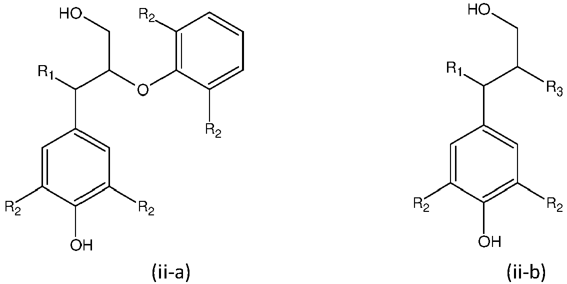

Wherein R₁: H or OSO₃M or SH
R₂: H or OCH₃
R₃: H, OCH₃
M: alkaline metal

Lignin can be any technical lignin such as Kraft lignin, lignosulfonate, organosolv lignin, or a combination thereof. In addition, the employed lignin can be fractionated or depolymerized by one of the following methods: base-catalyzed depolymerization, hydrogenolysis, pyrolysis, oxidation, acidolysis, or enzymatic degradation. The lignin may be further fractionated using membrane separation or solvent extractions. Ideally, lignin will have to be comprised out of oligomers and have a molecular weight between 100 and 100000 g/mol with a hydroxyl functionality between 0.1 and 25 mmol/g.

Lignin as a flame-retardant additive can be applied via three main processes: lignin-based surface coatings, melt-blending with polymers and chemically modified lignin. Chemical modification of lignin is typically done using P (phosphorus) modification; N (nitrogen) modification; P,N-modification without or with metal ion; silicon-containing and boron-containing modifications. Besides urea and N-alkoxyamines, melamine and its derivatives are the most well-known nitrogen-based class in this domain. Triazole derivatives, another nitrogen-rich compounds, have rarely been studied in combination with lignin, with only one known report using 3-amino-1,2,4-triazole for lignin modification. However, TAD (1,2,4-triazoline-3,5-dione) has never been developed so far for functionalization of lignin for improved fire-retardant properties. The ability of TAD to increase thermal stability has been highlighted on other material, such as poly(phosphoester) (PPE) polymers.

Lignin faced significant constraints to meet industry requirements when combined with a polymer matrix, with the main issue being a lack of homogeneity when blending/integrating lignin with polymeric matrices. To improve the fire retardancy (FR) performance of lignin, various advances have been taken, with the most efficient material being the modification of lignin with nitrogen and/or phosphorus molecules. However, almost all modifications so far are done using the OH (hydroxy) functional group of the lignin. This renders any further post-modification/integration into a new polymeric matrix extremely tedious. The herein invention functionalizes the lignin fragment at the ortho-position of the phenolic OH selectively using TAD derivatives as a reagent, leaving all the valuable OH functions still free for further reactions/integration into new polymer matrix such PU. This could be extended into any bioaromatic polymer bearing a phenol moiety bearing at least one unsubstituted ortho position. The tunability of the TAD reagent with different functionalities on the central nitrogen group also offers several opportunities for adapting the final lignin product towards any particular application.

### Summary of the invention

In a first aspect the present invention provides a urazole-functionalized lignin comprising the reaction product of a lignin, including the monomers of formulas (ii), (ii-a), (ii-b) and the depolymerized fragments derived of formula (iii) above, with a TAD (1,2,4-triazoline-3,5-dione) derivative according to formula (I) 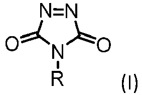 wherein R is hydrogen or any alkyl or aryl

The reaction product of a lignin with the TAD derivative will typically result in the functionalization at the ortho-position of the lignin's phenolic OH. Thus, in an embodiment according to the invention, it provides a urazole-functionalized lignin comprising the reaction product of a lignin, including the monomers of formulas (ii), (ii-a), (ii-b) and the depolymerized fragments derived of formula (iii) above, with a TAD (1,2,4-triazoline-3,5-dione) derivative according to formula (I) 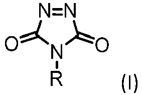 wherein R is hydrogen or any alkyl or aryl and wherein the lignin is urazole-functionalized at the ortho position of phenolic OH of the lignin.

Accordingly, in an embodiment the present invention provides a urazole-functionalized lignin comprising a repeating unit according to (II).

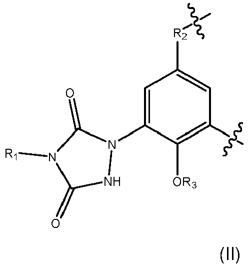

Wherein;
R₁ independently represents H, C₁₋₁₀alkyl, phenyl, substituted phenyl, benzyl or heteroaryl;
R₂ independently represents a direct bond, C ₁₋₆alkyl, or C ₂₋₆alkenyl, wherein each of said C ₁₋
₆alkyl, and C ₂₋₆alkenyl is independently and optionally substituted with from 1 to 3 substituents selected from OH, OSO₃M, SH and O-C₁₋₆alkyl.;
R₃ independently represents H, P(O)(OR₄)₂;
M represents an alkaline metal; and
R₄ represents H or the nitrogen atom from the amine of a nitrogen-containing flame retardant, such as e.g. ammonia, melamine, melem, melam, melon, melamine derivatives, melamine condensation products or melamine salts, benzoguanamine, polyisocyanurates, allantoin, (poly)phosphacenes, in particular melamine cyanurate, melamine phosphate, dimelamine phosphate, melamine pyrophosphate, melamine polyphosphate, melamine-methane phosphonate, melamine-metal phosphates, such as e.g. melamine aluminium phosphate, melamine zinc phosphate, melamine magnesium phosphate, and also the corresponding pyrophosphates and polyphosphates, ethylene diamine methane phosphate, poly-[2,4-(piperazin-1,4,-yl]-6-(morpholin-4-yl)3,5-triazine], ammonium polyphosphate, melamine borate, melamine hydrobromide, and the like.

In the aforementioned reaction the TAD derivative is a derivative according to formula (I) 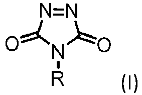 wherein R is hydrogen or any alkyl or aryl; in particular wherein R is H, C₁₋₁₀alkyl, phenyl, substituted phenyl, benzyl or heteroaryl; even more particular selected from phenyl-TAD (where R = Phenyl) or methyl-TAD (where R = Me).

In the urazole-functionalized lignin as herein provided, hereinafter also referred to as 'the material', the lignin is meant to include any technical lignin such as Kraft Lignin, lignosulfonate, organosolv lignin, or a combination thereof.

In an embodiment according to the invention, the lignin used in the manufacture of the material as herein provides is fractionated and/or depolymerized lignin oil, obtainable by one or more of the following methods: base-catalyzed depolymerization, hydrogenolysis, pyrolysis, oxidation, acidolysis, or enzymatic degradation.

In a particular embodiment, the lignin used in the manufacture of the material according to the invention is a fractionated and/or depolymerized lignin wherein said fractionated and/or depolymerized lignin is further fractionated using membrane separation or solvent extractions. In any of the aforementioned embodiments the lignin will typically comprise monomers, dimers, oligomers or a combination thereof. Hence, instead of identifying the lignin based on the method by which it is obtained, the lignin used in the manufacture of the material as herein provided, is meant to include a lignin composition comprising lignin derived monomers, dimers, oligomers or a combination of; in particular monomers, dimers, oligomers or combinations thereof with a molecular weight between 100 and 100000 g/mol; even more in particular a lignin composition comprising lignin derived monomers, dimers, oligomers or combinations thereof with a molecular weight between 100 and 100000 g/mol, and with a hydroxyl functionality between 0.1 and 25 mmol/g.

In a particular embodiment the lignin used in the manufacture of the material as herein provided, is meant to include a lignin composition comprising dimers, or oligomers having a repeating unit represented by formula (i-a) or (i-b) and/or comprising monomer represented by formula (ii) derived from the monolignols it is synthesized from, and /or repeating units derived from other link breakage or depolymerized fragments represented by formula (iii).

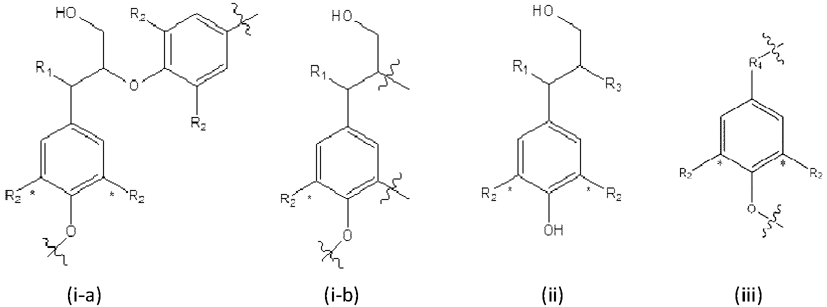

Wherein R₁: H or OSO₃M or SH
R₂: H or OCH₃
R₃: H, O-alkyl, or optionally substituted O-aryl
R₄: a direct bond, C ₁₋₆alkyl, or C ₂₋₆alkenyl, wherein each of said C ₁₋₆alkyl, and C ₂₋₆alkenyl is independently and optionally substituted with from 1 to 3 substituents selected from OH, OSO₃M, SH and O-C₁₋₆alkyl.
M: alkaline metal
* Indicates the ortho position vis-à-vis the phenolic OH of the lignin.

In a second aspect the present invention provides a method for obtaining a urazole-functionalized lignin as herein provided; said method comprising combining a TAD (1,2,4-triazoline-3,5-dione) derivative according to Formula (I) with a lignin as defined herein, in the presence or absence of a solvent, a base and a catalyst.

In an embodiment the base can be an organic or inorganic base, preferably a base such as alkoxides, hydroxides, carbonates, amines or a combination of.

In an embodiment the solvent is selected from water, alcohol, an organic solvent such as DCM, toluene, THF, DMF, DMSO, or a combination thereof; in particular an alcoholic solvent or water.

In another embodiment of the method for obtaining a urazole-functionalized lignin as herein provided; no solvent, and/or no base are used.

In a third aspect the present invention provides the use of the urazole-functionalized lignin as herein provided as flame retardant, as UV stabilizer, as antistatic or as antioxidant; more in particular as a flame-retardant additive. In such application as flame retardant, as UV stabilizer as antistatic or as antioxidant, the material can be used in accordance with common practice, such as for example via blending, condensation or reaction of the material with resins or polymers like polyamides, polyurethanes, and the like.

In a fourth aspect the present invention provides a method for integrating the urazole-functionalized lignin material as herein provided into a polymeric matrix via blending, condensation or reaction with said polymer for applications in flame retardants, UV stabilizers, polyolefins, polyamides, polyurethanes, antioxidant and resins.

### Detailed description of the invention

When describing the urazole-functionalized lignin material of the invention, the terms used are to be construed in accordance with the following definitions, unless a context dictates otherwise:
The term "alkyl" by itself or as part of another substituent refers to a fully saturated hydrocarbon of Formula CxH2x+1 wherein x is a number greater than or equal to 1. Generally, alkyl groups of this invention comprise from 1 to 20 carbon atoms; in particular from 1 to 10 carbon atoms; more in particular from 1 to 6 carbon atoms; even more particular from 1 to 4 carbon atoms. Alkyl groups may be linear or branched and may be substituted as indicated herein. When a subscript is used herein following a carbon atom, the subscript refers to the number of carbon atoms that the named group may contain. Thus, for example, C1-4alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. C1-C6 alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl.

The term "optionally substituted alkyl" refers to an alkyl group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

The term "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl.

The aryl ring, such as a phenyl ring, can optionally be substituted by one or more substituents. An "optionally substituted aryl" such as an optionally substituted phenyl refers to an aryl (e.g. phenyl) having optionally one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO2-NH2, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO2Ra, alkylthio, carboxyl, and the like, wherein Ra is alkyl or cycloalkyl.

Where a carbon atom in an aryl group is replaced with a heteroatom, the resultant ring is referred to herein as a heteroaryl ring.

The term "heteroaryl" as used herein by itself or as part of another group refers but is not limited to 5 to 12 carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, benzopyranyl, 1(4H)-benzopyranyl, 1(2H)-benzopyranyl, 3,4-dihydro-1(2H)-benzopyranyl, 3,4-dihydro-1(2H)-benzopyranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl, 7-azaindolyl, 6-azaindolyl, 5-azaindolyl, 4-azaindolyl.

As already mentioned herein before, in an embodiment the present invention is directed to a urazole-functionalized lignin comprising the reaction product of a lignin, including the monomers of formulas (ii), (ii-a), (ii-b) and the depolymerized fragments derived of formula (iii) above, with a TAD (1,2,4-triazoline-3,5-dione) derivative according to formula (I) 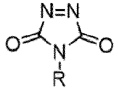
(I) wherein R is hydrogen or any alkyl or aryl and wherein the lignin is urazole-functionalized at the ortho position of phenolic OH of the lignin.

The urazole-functionalized polymeric lignin obtained, can generally be presented in comprising a repeating unit according to (II).

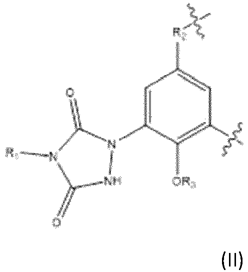

Wherein;
R₁ independently represents H, C₁₋₁₀alkyl, phenyl, substituted phenyl, benzyl or heteroaryl;
R₂ independently represents a direct bond, C ₁₋₆alkyl, or C ₂₋₆alkenyl, wherein each of said C ₁₋₆alkyl, and C ₂₋₆alkenyl is independently and optionally substituted with from 1 to 3 substituents selected from OH, OSO₃M, SH and O-C₁₋₆alkyl.;
R₃ independently represents H, P(O)(OR₄)₂;
M represents an alkaline metal; and
R₄ represents H or the nitrogen atom from the amine of a nitrogen-containing flame retardant, such as e.g. ammonia, melamine, melem, melam, melon, melamine derivatives, melamine condensation products or melamine salts, benzoguanamine, polyisocyanurates, allantoin, (poly)phosphacenes, in particular melamine cyanurate, melamine phosphate, dimelamine phosphate, melamine pyrophosphate, melamine polyphosphate, melamine-methane phosphonate, melamine-metal phosphates, such as e.g. melamine aluminium phosphate, melamine zinc phosphate, melamine magnesium phosphate, and also the corresponding pyrophosphates and polyphosphates, ethylene diamine methane phosphate, poly-[2,4-(piperazin-1,4,-yl]-6-(morpholin-4-yl)3,5-triazine], ammonium polyphosphate, melamine borate, melamine hydrobromide, and the like.

General scheme of the TAD modification reaction of a lignin or lignin-derived monomers, dimers and oligomers

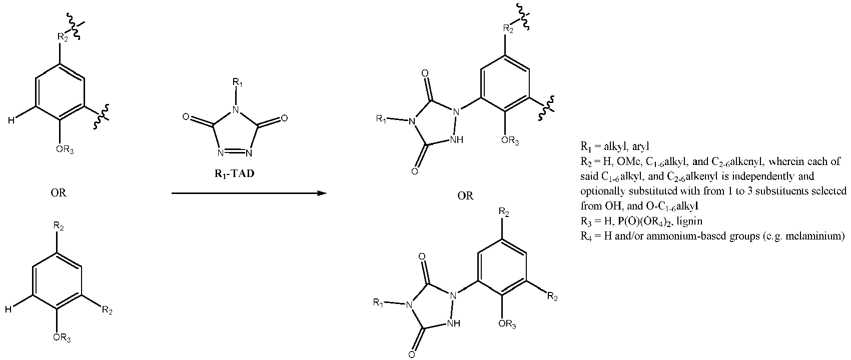

The TAD selectively adds to the ortho-position of the phenolic moiety of lignin leaving the OH groups intact and ready for further functionalization. This was demonstrated in this invention by further modifying the TAD-lignin product with P,N modification.

In order to perform this reaction, the lignin used need to have at least one unsubstituted ortho-position on the phenolic moiety, which is determined by the total amount of uncondensed phenolic OH (mmol/g). The higher the latter amount is, the more unsubstituted phenolic ortho-positions are available for functionalization by the TAD.

### Examples

In the below examples, the functionalization of lignin was first tested using PTAD (phenyl-TAD, where R = Phenyl) with lignin derived monomers, hereinafter also referred to as the model compounds. These model compounds are known to be produced from lignin either in pure form or as mixtures with other monomers, dimers and oligomers (for more details, see: Current Opinion in Green and Sustainable Chemistry, 2023, 40, 100781). After finding the optimized reaction condition, the next step was functionalization of different lignin samples under the same condition. Two types of lignin were used in this invention: lignin oil from in house depolymerized process and lignin solid extracted from the Kraft lignin.

For further development to improve the flame retardancy of lignin, P,N-modification was considered. These modifications were first carried out on the model compounds, then on the lignin samples. Here, the functionalized lignin and non-functionalized lignin were used to figure out the importance of functionalization by TAD moiety on changing the flame-retardant properties of lignin.

### Materials and Methods

Reagents and solvents are commercially available and used without further purification.
- The model compounds 4-propylguaiacol (PG) (CAS: 2785-87-7), dihydroconiferyl alcohol (DCA) (CAS: 2305-13-7) and the TAD derivative: 4-phenyl-1,2,4-triazoline-3,5-dione (PTAD) (CAS: 4233-33-4) are commercially available.
- The dimer PGD was synthesized in SPOT group.
- The lignin samples **(LO 1; LO 2** and **LS)** were produced in SPOT group.

### Methods

- Flash chromatography was performed using silica gel 60, particle size 40-63 µm.
- NMR spectra were recorded in deuterated solvents on an 80 MHz Spinsolve Ultra from Magritek.
- IR spectra were recorded by a Nicolet iS10 FT-IR spectrometer.
- TGA measurements were recorded on a NETZSCH STA 449C machine.
- Elemental analysis were recorded on a Vario El Cube from Elementar.
- Cone calorimetry (Heat release rate measurements done using a mass loss calorimeter)

### Examples of functionalization of the model compounds using PTAD

### Example 1:

To a round-bottom flask containing a solution of 4-propylguaiacol (PG) (63.0 mg, 0.38 mmol, 1 equiv) in MeOH (3 mL) was added NaO^{t}Bu (43.7 mg, 0.46 mmol, 1.2 equiv) by portion. The reaction mixture was stirred at room temperature for 30 minutes. After 30 minutes, the flask was covered by aluminum foil and the reaction was stirred in the dark fume hood. Then PTAD (99.7 mg, 0.57 mmol, 1.5 equiv) was added. The reaction was stirred at room temperature for 3 hours. After 3 hours, the reaction mixture was diluted by EtOAc, followed by the addition of HCl (10%). The reaction mixture was extracted from EtOAc. The aqueous phase was washed with EtOAc. The combined organic phase was washed with brine, dried with MgSO₄ and then concentrated under reduced pressure. The residue was purified by flash chromatography (SiO₂, MeOH/CH₂Cl₂ = 0/100 then 1/100) to afford the target compound as a white foam (120 mg, 93% yield). The formation of the product was confirmed by ¹H NMR (80 MHz, CDCl₃).

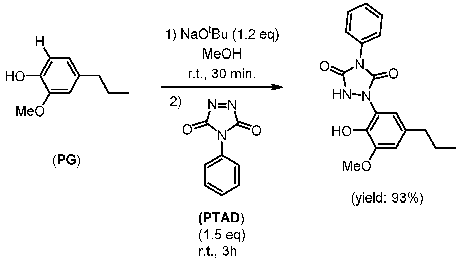

### Example 2:

To a round-bottom flask containing a solution of dihydroconiferyl alcohol (DCA) (60.0 mg, 0.33 mmol, 1 equiv) in MeOH (3 mL) was added NaO^{t}Bu (75.9 mg, 0.79 mmol, 2.4 equiv) by portion. The reaction mixture was stirred at room temperature for 30 minutes. After 30 minutes, the flask was covered by aluminum foil and the reaction was stirred in the dark fume hood. Then PTAD (86.5 mg, 0.49 mmol, 1.5 equiv) was added. The reaction was stirred at room temperature for 3 hours. After 3 hours, the reaction mixture was diluted by EtOAc, followed by the addition of HCl (10%). The reaction mixture was extracted from EtOAc. The aqueous phase was washed with EtOAc. The combined organic phase was washed with brine, dried with MgSO₄ and then concentrated under reduced pressure. The residue was purified by flash chromatography (SiO₂, MeOH/CH₂Cl₂ = 0/100 then 4/100) to afford the target compound as an off-white foam (55 mg, 47% yield). The formation of the product was confirmed by ¹H NMR (80 MHz, CDCl₃).

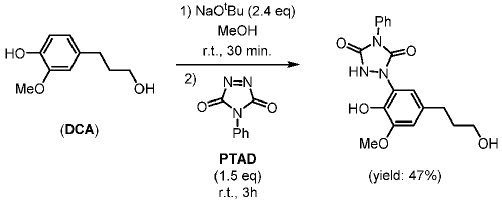

### Example 3:

To a round-bottom flask containing a solution of PGD (69.0 mg, 0.20 mmol, 1 equiv) in MeOH (3 mL) was added NaO^{t}Bu (46.1 mg, 0.48 mmol, 2.4 equiv) by portion. The reaction mixture was stirred at room temperature for 30 minutes. After 30 minutes, the flask was covered by aluminum foil and the reaction was stirred in the dark fume hood. Then PTAD (105.1 mg, 0.60 mmol, 3 equiv) was added. The reaction was stirred at room temperature for 3 hours. After 3 hours, the reaction mixture was diluted by EtOAc, followed by the addition of HCl (10%). The reaction mixture was extracted from EtOAc. The aqueous phase was washed with EtOAc. The combined organic phase was washed with brine, dried with MgSO₄ and then concentrated under reduced pressure. The residue was purified by flash chromatography (SiO₂, MeOH/CH₂Cl₂ = 0/100 then 4/100) to afford the target compound as a white solid (83 mg, 60% yield). The formation of the product was confirmed by ¹H NMR (80 MHz, DMSO-*d*₆).

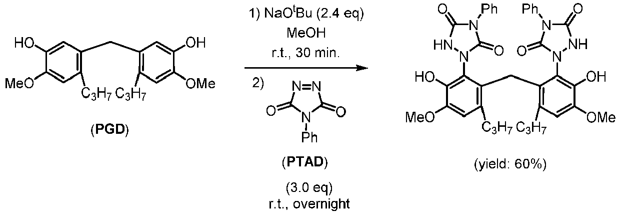

### Examples of P-modification of the model compounds and the TAD-modified PG

### Example 4:

The glassware used for this reaction was connected to the Schlenk line and dried under vacuum using a heat gun. The reaction was carried out in a dry condition and in N₂ atmosphere. Triethylamine TEA (2.10 mL, 15.16 mmol, 2 equiv) and anhydrous THF (3 mL) were added into a three-necked flask equipped with a condenser. The solution was cooled down to 0 °C using an ice bath. Then POCl₃ (0.92 mL, 9.85 mmol, 1.3 equiv) was added slowly. Thereafter, a solution of PG (1.21 mL, 7.58 mmol, 1 equiv) in THF (3 mL) was added slowly at 0 °C. The reaction was warmed up gradually to room temperature and stirred overnight. The reaction mixture was then cooled down to 0 °C and quenched with water. The mixture was extracted with Et₂O. The combined organic layers were washed with brine, dried with MgSO₄ and concentrated under reduced pressure. The product was obtained as a yellow transparent oil (quantitative yield).

The successful phosphorylation was confirmed by ¹H and ³¹P NMR spectra as well as IR spectrum. The product was pure enough for the next step without any further purification.

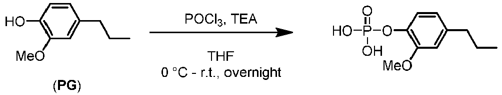

### Example 5:

The glassware used for this reaction was connected to the Schlenk line and dried under vacuum using a heat gun. The reaction was carried out in a dry condition and in N₂ atmosphere. Triethylamine TEA (1.65 mL, 11.85 mmol, 4 equiv) and anhydrous THF (3 mL) were added into a three-necked flask equipped with a condenser. The solution was cooled down to 0 °C using an ice bath. Then POCl₃ (0.69 mL, 7.41 mmol, 2.5 equiv) was added slowly. Thereafter, a solution of DCA (540 mg, 2.96 mmol, 1 equiv) in THF (3 mL) was added slowly at 0 °C. The reaction was warmed up gradually to room temperature and stirred overnight. The reaction mixture was then cooled down to 0 °C and quenched with water. The mixture was extracted with EtOAc. The combined organic layers were washed with brine, dried with MgSO₄ and concentrated under reduced pressure. The product was obtained as a yellow transparent oil.

TLC as well as ¹H and ³¹P NMR spectra showed that DCA as the starting material was consumed totally, the mono-phosphorylated product was obtained as the major one and can be used for the next step without any further purification.

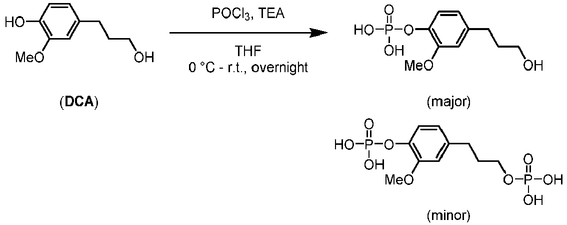

### Example 6:

The glassware used for this reaction was connected to the Schlenk line and dried under vacuum using a heat gun. The reaction was carried out in a dry condition and in N₂ atmosphere. Triethylamine TEA (0.24 mL, 1.73 mmol, 2 equiv) and anhydrous THF (5 mL) were added into a three-necked flask equipped with a condenser. The solution was cooled down to 0 °C using an ice bath. Then POCl₃ (0.11 mL, 1.13 mmol, 1.3 equiv) was added slowly. Thereafter, a solution of TAD-modified PG (296 mg, 0.87 mmol, 1 equiv) which is the product in the example 1 in THF (1.5 mL) was added slowly at 0 °C. The reaction was warmed up gradually to room temperature and stirred overnight. The reaction mixture was then cooled down to 0 °C and quenched with water. The mixture was extracted with Et₂O. The combined organic layers were washed with brine, dried with MgSO₄ and concentrated under reduced pressure. The product was obtained as an off-white solid (260 mg, 71% yield).

The successful phosphorylation was confirmed by ¹H and ³¹P NMR spectra, IR spectrum and elemental analysis. The product was pure enough for the next step without any further purification.

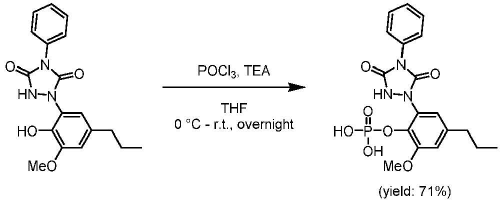

### Examples of P,N-modification of the model compounds and the TAD-modified PG

### Example 7:

The product of phosphorylation shown in example 4 was used as the staring material for this reaction. A mixture of the latter (866 mg, 7.58 mmol) and melamine (1912 mg, 15.16 mmol, 2 equiv) in THF (20 mL) was stirred at 60 °C overnight. THF was then removed under reduced pressure. The solid residue was washed with MeOH to remove the free melamine left. The product was obtained as a white solid after drying under vacuum.

The successful P,N-modification was confirmed by ¹H and ³¹P NMR spectra, IR spectrum and elemental analysis.

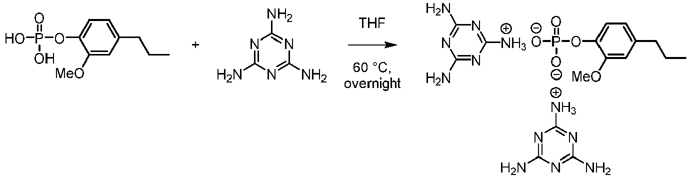

### Example 8:

The product of phosphorylation shown in example 5 was used as the staring material for this reaction. A mixture of the latter (320 mg) and melamine (472 mg, 3.74 mmol) in THF (10 mL) was stirred at 60 °C overnight. THF was then removed under reduced pressure. The solid residue was washed with MeOH to remove the free melamine left. The product was obtained as an off-white solid after drying under vacuum.

The successful P,N-modification was confirmed by ¹H and ³¹P NMR spectra, IR spectrum and elemental analysis.

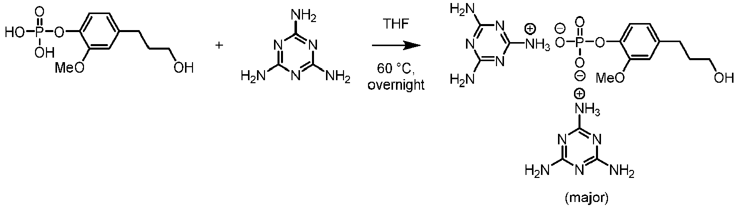

### Example 9:

The product of phosphorylation shown in example 6 (254 mg, 0.60 mmol) was used as the staring material for this reaction. A mixture of this compound and melamine (152 mg, 1.21 mmol, 2 equiv) in THF (10 mL) was stirred at 60 °C overnight. THF was then removed under reduced pressure. The solid residue was washed with MeOH to remove the free melamine left. The product was obtained as a white solid after drying under vacuum.

The successful P,N-modification was confirmed by ¹H and ³¹P NMR spectra, IR spectrum and elemental analysis.

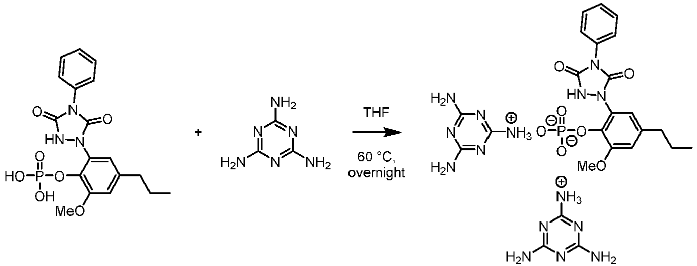

### Examples of lignin functionalization using PTAD

The lignin samples used in this invention are the lignin oil **(LO 1)** and **(LO 2)** from depolymerization process and the lignin solid **(LS)** extracted from Kraft lignin. These lignin samples were produced in house.

In order to calculate the amount of sodium tert-butoxide (NaO^{t}Bu) and PTAD needed for lignin functionalization, the total amount of the uncondensed phenolic OH and of the OH groups (in mmol/g) in the lignin samples were determined by ³¹P NMR method shown in the known procedure as follows:
_X. Meng, C. Crestini, H. Ben, N. Hao, Y. Pu, A. J. Ragauskas, D. S. Argyropoulos, Nature Protocols, 2019, 14, 2627-2647
_J. Gracia-Vitoria, M. Rubens, E. Feghali, Peter Adriaensens, K. Vanbroekhoven, R. Vendamme, Industrial Crops & Products, 2022, 176, 114405

The results are shown in Table 1.

**Table 1. the OH value in the lignin samples**

| **Sample** | **Aliphatic OH (mmol/g) (145.4-150 ppm)** | **Condensed Phenolic OH (mmol/g) (140-144.5 ppm)** | **Uncondensed Phenolic OH (mmol/g) (137.6-140.2 ppm)** | | | **Total Uncondensed Phenolic OH (mmol/g)** | **Total Phenolic OH (mmol/g** | **Carboxylic acid (mmol/g) (133.6-136 ppm)** |
|---|---|---|---|---|---|---|---|---|
| | | | Guaiacyl (139-140.2 ppm) | Catechol (138.9 ppm) | p-hydroxylphenyl (137.8 ppm) | | | |
| **LO 1** | 0.73 | 0.24 | 0.36 | 0.00 | 0.09 | 0.45 | 0.69 | 0.23 |
| **LO 2** | 2.27 | 0.63 | 2.43 | 0.16 | 0.16 | 2.67 | 3.30 | 0.16 |
| **LS** | 2.55 | 2.00 | | | | 2.76 | 4.76 | 0.45 |

### Example 10:

To a round-bottom flask containing a solution of the lignin oil **(LO 1)** (1000 mg, 0.45 mmol/g of the total uncondensed OH, 1.65 mmol/g of the total OH) in MeOH (10 mL) was added NaO^{t}Bu (190 mg, 1.98 mmol) by portion. The reaction mixture was stirred at room temperature for 30 minutes. Then PTAD (118 mg, 0.68 mmol) was added. The reaction was stirred at room temperature for 3 hours. After 3 hours, the reaction mixture was diluted by EtOAc, followed by the addition of HCl (10%). The reaction mixture was extracted from EtOAc. The aqueous phase was washed with EtOAc. The combined organic phase was washed with brine, dried with MgSO₄ and then concentrated under reduced pressure to afford the product as a dark brown oil.

The successful functionalization of lignin by using PTAD was confirmed by ¹H NMR and C, H, N elemental analysis. Having already a low amount of total Uncondensed Phenolic OH (Table 1, 0.45 mmol/g) meant that low amounts of TAD reacted but still the degree of functionalization (48%) was quite acceptable.

| **Sample** | **Elemental analysis (in %)** | | |
|---|---|---|---|
| | C | H | N |
| **LO 1 (before PTAD modification)** | 74.6 | 11.8 | **3.4** |
| **LO 1 (after PTAD modification)** | 64.0 | 9.7 | **4.3** |

### Example 11:

To a round-bottom flask containing a solution of the lignin oil **(LO 2)** (307 mg, 2.67 mmol/g of the total uncondensed OH, 5.73 mmol/g of the total OH) in MeOH (10 mL) was added NaO^{t}Bu (220 mg, 2.29 mmol) by portion. The reaction mixture was stirred at room temperature for 30 minutes. Then PTAD (215 mg, 1.23 mmol) was added. The reaction was stirred at room temperature for 3 hours. After 3 hours, the reaction mixture was diluted by EtOAc, followed by the addition of HCl (10%). The reaction mixture was extracted from EtOAc. The aqueous phase was washed with EtOAc. The combined organic phase was washed with brine, dried with MgSO₄ and then concentrated under reduced pressure to afford the product as a dark brown oil.

The successful functionalization of lignin by using PTAD was confirmed by ¹H NMR and C, H, N elemental analysis. Degree of functionalization was calculated to be 79%.

| **Sample** | **Elemental analysis (in %)** | | |
|---|---|---|---|
| | C | H | N |
| **LO 2 (before PTAD modification)** | 65.8 | 7.6 | **0.7** |
| **LO 2 (after PTAD modification)** | 56.9 | 6.3 | **9.6** |

### Example 12:

*Lignin solid (LS) extraction from Kraft lignin:* In a 2 L round bottom flask equipped with mechanical stirring, 800 g of technical MeOH were added to 200 g of Kraft lignin powder. The mixture was stirred at 150 rpm and 50 °C for 5 hours. Later, the mixture was separated by gravity filtration using a filter paper to remove the insoluble fraction. The excess of methanol from the lignin soluble mixture was removed in the rotary. The dark solid chunks were scratched from the walls of the flask, grinded and keep it in the vacuum oven at 45 °C overnight (16 h). The final product was characterized via ³¹P NMR, FT-IR and GPC (82-84 g, 41-42%).

*TAD reaction with LS:* To a round-bottom flask containing a solution of the lignin solid (LS) (6.00 g, 2.76 mmol/g of the total uncondensed OH, 7.76 mmol/g of the total OH) in MeOH (133 mL) was added NaO^{t}Bu (5.37 g, 55.9 mmol) by portion. The reaction mixture was stirred at room temperature for 30 minutes. Then PTAD (4.35 g, 24.8 mmol) was added. The reaction was stirred at room temperature for 3 hours. After 3 hours, the reaction mixture was concentrated under reduced pressure to remove MeOH. The residue was dissolved in EtOAc, followed by the addition of HCl (1M). The mixture was extracted from EtOAc. The aqueous phase was washed with EtOAc. The combined organic phase was dried with MgSO₄ and then concentrated under reduced pressure to afford the product **(Urazole-LS)** as a brown solid.

The successful functionalization of lignin by using PTAD was confirmed by ¹H NMR and C, H, N elemental analysis. Degree of functionalization was calculated to be >68%.

| **Sample** | **Elemental analysis (in %)** | | |
|---|---|---|---|
| | C | H | N |
| **LS (before PTAD modification)** | 64.9 | 6.5 | **<0.2** |
| **LS (after PTAD modification)** | 59.9 | 5.4 | **8.1** |

### Example 13:

The same lignin sample **(LS)** and the TAD reaction procedure as in the example 12 were repeated on a 24 grams scale of the lignin solid **(LS).** The corresponding product **(Urazole-LS)** is a brown solid.

### Examples of P-modification of the lignin sample

### Example 14:

The glassware used for this reaction was connected to the Schlenk line and dried under vacuum using a heat gun. The reaction was carried out in a dry condition and in N₂ atmosphere. To a three-necked flask equipped with a condenser was added the lignin solid (LS) (20 g, 7.76 mmol/g of the total OH). Then anhydrous THF (200 mL) was added. The mixture was cooled down to 0 °C using an ice bath. Triethylamine TEA (43.3 mL, 310 mmol, 2 equiv) was added, followed by the slow addition of POCl₃ (18.8 mL, 202 mmol, 1.3 equiv) at 0 °C. The reaction mixture was warmed up gradually to room temperature and stirred overnight. The reaction was then cooled down to 0 °C and quenched with water. The reaction mixture was filtered and the solid product was washed several times with water. The product was obtained as a yellow solid after drying under vacuum and was used directly for the next step.

The successful phosphorylation was confirmed by IR spectrum and elemental analysis.

### Example 15:

The glassware used for this reaction was connected to the Schlenk line and dried under vacuum using a heat gun. The reaction was carried out in a dry condition and in N₂ atmosphere. To a three-necked flask equipped with a condenser was added the PTAD modified lignin solid **(Urazole-LS)** (the reaction product from example 13 (22.3 g, 7.76 mmol/g of the total OH). Then anhydrous THF (200 mL) was added. The mixture was cooled down to 0 °C using an ice bath. Triethylamine TEA (48.3 mL, 346 mmol, 2 equiv) was added, followed by the slow addition of POCl₃ (21 mL, 225 mmol, 1.3 equiv) at 0 °C. The reaction mixture was warmed up gradually to room temperature and stirred overnight. The reaction was then cooled down to 0 °C and quenched with water. The reaction mixture was filtered and the solid product was washed several times with water. The product was obtained as a yellow solid after drying under vacuum and was used directly for the next step.

The successful phosphorylation was confirmed by IR spectrum and elemental analysis.

### Examples of P,N-modification of the lignin sample

### Example 16:

The product of phosphorylation shown in example 14 was used as the starting material for this reaction. A mixture of the latter and melamine (39.15 g, 310.4 mmol, 2 equiv) in THF (500 mL) was stirred at 60 °C overnight. THF was then removed under reduced pressure. The solid residue was washed with MeOH, then several times with water until no free melamine left. The disappearance of free melamine was checked by IR spectrum. The product was obtained as a yellow solid after drying under vacuum.

The successful P,N-modification was confirmed by IR spectrum and elemental analysis.

### Example 17:

The product of phosphorylation shown in example 15 was used as the starting material for this reaction. A mixture of the latter (18.5 g) and melamine (36.5 g, 289.5 mmol, 2 equiv) in THF (500 mL) was stirred at 60 °C overnight. THF was then removed under reduced pressure. The solid residue was washed with MeOH, then several times with water until no free melamine left. The disappearance of free melamine was checked by IR spectrum. The product was obtained as a yellow solid after drying under vacuum.

The successful P,N-modification was confirmed by IR spectrum and elemental analysis.

### Results and conclusions

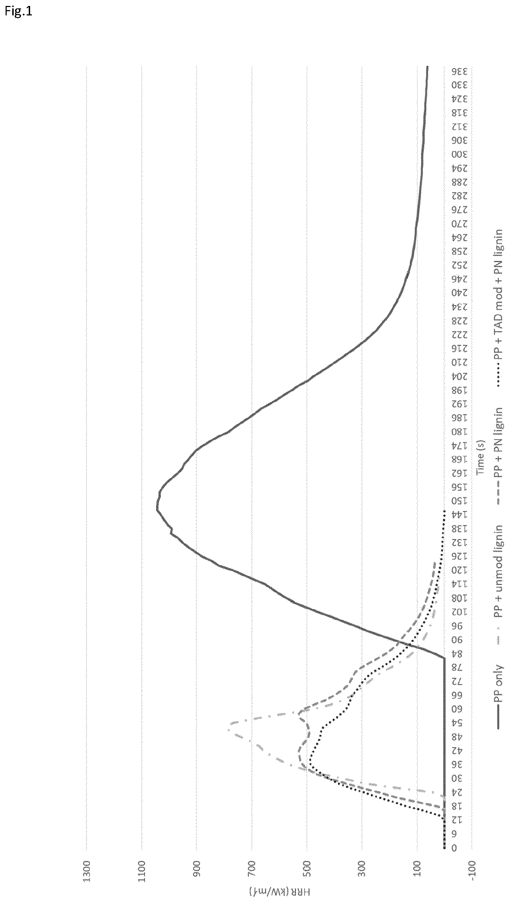

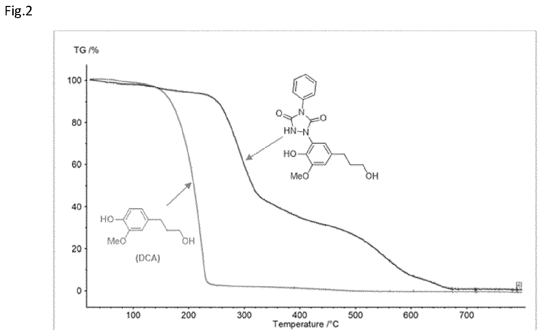

The functionalization using PTAD worked well not only on the model compounds (from moderate to excellent yield) but also on the target lignin. In this invention, the depolymerized lignin oil (LO 1 and LO 2) as well as the lignin solid (LS) extracted from Kraft lignin were functionalized successfully with the degree of functionalization of 48%, 79% and 68%, respectively. This shows that the process can be applied for different types of lignin. In addition, the procedure is reproducible from small scale to large scale (60 mg scale to 24-gram scale). The importance of TAD skeleton on increasing thermal stability was also proved by cone calorimetry (Figure 1) and TGA measurements (see example in Figure 2).

For further improvement, P,N-modification were carried out. This two-step synthesis worked perfectly on both non-functionalized and PTAD-functionalized model compounds as well as lignin sample (LS) from small to large scale (250 mg scale to 20-gram scale).

In Figure 1 (Heat release rate (HRR) results (using cone calorimetry)), polypropylene (PP) + 20% lignin blends were prepared for different lignin samples to perform cone calorimeter tests, and compare the effect of the lignin additives on the heat release rate (HRR) of the blended polymer material. The PP reference chosen is PP PHC 26 from SABIC (MFR @230 °C, 2,16 kg = 8 g/10 min). Three different samples of lignin were compared: unmodified lignin (unmod lignin), lignin modified with P,N (PN lignin) and lignin modified with P,N and with TAD (TAD mod + PN lignin).

Cone calorimetry showed the typical effect of lignin to reduce the time to ignition (TTI) and total heat release rate (HRR). The PP-unmodified lignin sample had a high HRR peak of around 800 KW/m². The one with P,N modified lignin improves on the initial material by lowering the peak HRR to ~520 KW/m². The one with P,N and TAD modified lignin gave the best result by lowering the peak HRR below 490 KW/m². The latter material also shows the most decrease in the TTI and the lowest overall HRR, highlighting the significant impact TAD modification has on the lignin sample.

In Figure 2 (TGA results before and after TAD modification of DCA) illustrates the effect of TAD skeleton on increasing thermal stability of lignin derived compounds.

## Claims

1. A urazole-functionalized lignin comprising the reaction product of a lignin with a TAD (1,2,4-triazoline-3,5-dione) derivative according to formula (I) 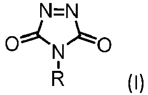 wherein R is any alkyl or aryl and wherein the lignin is urazole-functionalized at the ortho position of phenolic OH of the lignin.

2. A urazole-functionalized lignin according to claim 1, comprising a repeating unit according to (II). 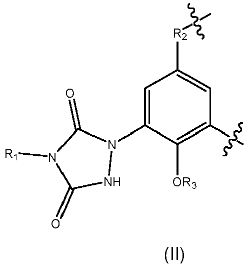 Wherein;
R₁ independently represents H, C₁₋₁₀alkyl, phenyl, substituted phenyl, benzyl or heteroaryl;
R₂ independently represents a direct bond, C ₁₋₆alkyl, or C ₂₋₆alkenyl, wherein each of said C ₁₋₆alkyl, and C ₂₋₆alkenyl is independently and optionally substituted with from 1 to 3 substituents selected from OH, OSO₃M, SH and O-C₁₋₆alkyl.;
R₃ independently represents H, P(O)(OR₄)₂;
M represents an alkaline metal; and
R₄ represents H or the nitrogen atom from the amine of a nitrogen-containing flame retardant, such as e.g. ammonia, melamine, melem, melam, melon, melamine derivatives, melamine condensation products or melamine salts, benzoguanamine, polyisocyanurates, allantoin, (poly)phosphacenes, in particular melamine cyanurate, melamine phosphate, dimelamine phosphate, melamine pyrophosphate, melamine polyphosphate, melamine-methane phosphonate, melamine-metal phosphates, such as e.g. melamine aluminium phosphate, melamine zinc phosphate, melamine magnesium phosphate, and also the corresponding pyrophosphates and polyphosphates, ethylene diamine methane phosphate, poly-[2,4-(piperazin-1,4,-yl]-6-(morpholin-4-yl)3,5-triazine], ammonium polyphosphate, melamine borate, melamine hydrobromide, and the like.

3. The material of claim 1 wherein the TAD derivative is selected from R-TAD according to formula (I) wherein R is hydrogen or any alkyl or aryl; in particular wherein R is H, C₁₋₁₀alkyl, phenyl, substituted phenyl, benzyl or heteroaryl; even more particular selected from phenyl-TAD (where R = Phenyl) or methyl-TAD (where R = Me).

4. The material of claim 1 wherein the lignin is any technical lignin such as Kraft Lignin, lignosulfonate, organosolv lignin, or a combination thereof.

5. The material according to any one of the preceding claims wherein the lignin is fractionated and/or depolymerized by one of the following methods: base-catalyzed depolymerization, hydrogenolysis, pyrolysis, oxidation, acidolysis, or enzymatic degradation.

6. The material according to any one of the preceding claims wherein the lignin is further fractionated using membrane separation or solvent extractions.

7. The material according to any one of the preceding claims wherein the lignin is comprised out of monomers, dimers, oligomers or a combination of, and have a molecular weight between 100 and 100000 with a hydroxyl functionality between 0.1 and 25 mmol/g.

8. A method for obtaining a urazole-functionalized lignin according to claim 1; said method comprising combining a TAD (1,2,4-triazoline-3,5-dione) derivative according to Formula (I) with a lignin as defined in any one of claims 4 to 7, in the presence or absence of a solvent, a base and a catalyst.

9. The method according to claim 8, wherein the base can be an organic or inorganic base, preferably a base such as alkoxides, hydroxides, carbonates, amines or a combination of.

10. The method according to claim 8, wherein the solvent is water, alcohol, an organic solvent such as DCM, toluene, THF, DMF, DMSO, or a combination thereof; in particular an alcoholic solvent or water.

11. The method according to preceding claims, wherein no solvent, and/or no base are used.

12. A method for integrating the material according to claim 1 into a polymeric matrix via blending, condensation or reaction with said polymer for applications in flame retardants, UV stabilizers, antistatic, polyolefins, polyamides, polyurethanes, antioxidant and resins.
